# EUROPÄISCHE PATENTSCHRIFT

(11) **EP 0 413 981 B1**
(45) Veröffentlichungstag und Bekanntmachung des Hinweises auf die Patenterteilung: **01.06.1994**
(21) Anmeldenummer: 90114128.3
(22) Anmeldetag: 24.07.1990
(51) Int. Cl.: C07D 251/38, C02F 1/62

(54) **Dinatriumsalz des Trimercapto-s-triazin-hexahydrats, Verfahren zu seiner Herstellung und Verwendung**
Dinatriumsalt of the trimercapto-s-triazine-hexahydrate, method for its preparation and its use
Sel disodique de hexahydrate de trimercapto-s-triazine, procédé pour sa préparation et utilisation

(30) Priorität: 19.08.1989 DE 3927469
(43) Veröffentlichungstag der Anmeldung: 27.02.1991
(73) Patentinhaber: Degussa Aktiengesellschaft, 60311 Frankfurt (DE)
(72) Erfinder: Hentschel, Klaus, Dr., D-6458 Rodenbach (DE); Samson, Marc, Dr., B-9100 Lokeren (BE); Vingerhoets, Marcel, B-2168 Brecht (BE); Weber, Karl-Ludwig, Dr., B-2080 Kapellen (BE)

(56) Entgegenhaltungen:
- DE-A- 3 729 029
- CHEMICAL ABSTRACTS, Band 100, Nr. 6, 6. Februar 1984, Seite 43, Spalte 2, Zusammenfassung-Nr. 35 469v, Columbus, Ohio, USA; JAPAN SYNTHETIC RUBBER CO.: "Lamination of halogenated polymers with diene rubbers"
- CHEMICAL ABSTRACTS, Band 99, Nr. 24, 12. Dezember 1983, Seite 46, Spalte 2, Zusammenfassung-Nr. 196 068q, Columbus, Ohio, USA; JAPAN SYNTHETIC RUBBER CO.: "Co-crosslinked polymer composition obtained from halogen-containing polymer and unsaturated polymer"
- CHEMICAL ABSTRACTS, Band 102, Nr. 2, 14. Januar 1985, Seite 66, Spalte 1, Zusammenfassung-Nr. 7 996n, Columbus, Ohio, USA; Y. NAKAMURA et al.: "Studies on crosslinking adhesion. II. Crosslinking adhesion of fluoro rubber to nitrile or epichlorohydrin rubber"

## Beschreibung

Die Erfindung betrifft das Dinatriumsalz des Trimercapto-s-triazin-hexahydrats, ein Verfahren zu seiner Herstellung und seine Verwendung. Die Verbindung weist folgende Strukturformel auf 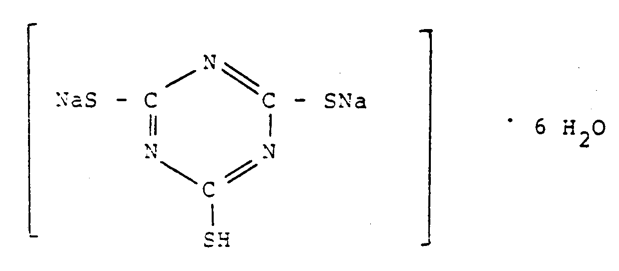
und wird nachfolgende als TMT-Na₂ · 6 H₂O bezeichnet.

Trimercapto-s-triazin, nachfolgend TMT-H₃ genannt, sowie Natriumsalze dieser dreiwertigern Säure wurden bereits von A.W. Hofmann beschrieben - Chem. Ber. 18 (1885), 2196-2207. Durch Umsetzung von 2,4,6-Trichlor-s-triazin (Cyanurchlorid) mit Natriumsulfid konnte nach Ansäuern TMT-H₃ gewonnen werden. Seinerzeit konnte auch ein Mononatriumsalz des Trimercapto-s-triazins isoliert werden.

Nakamura et al. - Japan Kokai 49/1580 (Chem. Abstr. 81, 3972 b) - gelang die Herstellung des Mononatriumsalzes von Trimercapto-s-triazin in Form des Trihydrates (TMT-Na · 3 H₂O) durch Umsetzung von NaHS und Na₂S mit Cyanurchlorid in wäßriger Phase.

Gemäß DE-AS 22 40 549 lassen sich unter Verwendung von Trimercapto-s-triazin oder seinen wasserlöslichen Alkalimetallsalzen Schwermetalle, wie z. B. Cu, Cd, Ni, Hg, Ag, Pb, als schwerlösliche Verbindungen aus Abwässern abtrennen. Zur Anwendung kommt in den beispielhaften Ausführungen dieses Dokuments bevorzugt festes TMT-Na · 3 H₂O oder eine 3 gew.-%ige wäßrige Lösung des Mononatriumsalzes des Trimercapto-s-triazins (TMT-Na). Das Di- und Trinatriumsalz von Trimercapto-s-triazin, nachfolgend TMT-Na₂ bzw. TMT-Na₃ genannt, werden gleichfalls als mögliche Fällungsreagenzien vorgeschlagen. Die Sättigungskonzentration wäßriger Lösungen der Alkalimetall-, Ammonium- und Erdalkalimetallsalze von Mono-, Di- und Trimercapto-s-triazinen wird mit 0,01 bis 25 Gew.-% bei 25 °C angegeben. Aus der DE-AS 22 40 733 ist ein Verfahren zur Fixierung von Schwermetallionen im Erdreich zu entnehmen, bei dem u. a. auch eine wäßrige Lösung des Trimercapto-s-triazin-trinatriumsalzes (TMT-Na₃) verwendet wird. Hinweise über die Herstellung von TMT-Na₂ und TMT-Na₃ sowie deren Löslichkeit in Wasser sind den beiden Dokumenten nicht zu entnehmen.

Wäßrige Lösungen von TMT-Na₃ sind im Handel erhältlich und finden insbesondere Anwendung zur Schwermetallabtrennung aus Rauchgaswaschwässern von Kraftwerken und Müllverbrennungsanlagen, aus Abwässern der Minenindustrie sowie galvanotechnischer und chemischer Betriebe. Die im Handel erhältliche wäßrige TMT-Na₃-Lösung weist eine Konzentration von 15 Gew.-% auf (Firmenschrift TMT 15 der Fa. Degussa 3/1986). Die Sättigungskonzentration von TMT-Na₃ in Wasser beträgt bei 0 °C etwa 16 Gew.-%.

Wie aus der DE-OS 37 29 029 hervorgeht, konnte erstmals vor kurzem TMT-Na₃ als Nonahydrat auch in kristalliner Form gewonnen werden. Die Herstellung von TMT-Na₃ · 9 H₂O umfaßt die Stufen Umsetzung von Cyanurchlorid mit NaHS oder Na₂S oder einem NaHS/Na₂S-Gemisch in wäßrigem Medium bei einem pH-Wert oberhalb von 7 bei 20-70 °C, anschließend Einstellung des pH-Wertes auf vorzugsweise um 12,5 und Abkühlen des Reaktionsgemisches, wobei TMT-Na₃ · 9 H₂O auskristallisiert. Nach dem Trocknen enthält das Produkt 60 Gew.-% TMT-Na₃ und 40 Gew.-% Kristallwasser.

Wie bereits oben vermerkt, ist bisher kein Dokument bekannt geworden, das ein Dinatriumsalz des Trimercapto-s-triazins in fester Form beschreibt.

Gegenstand der Erfindung ist somit das Dinatriumsalz des Trimercapto-s-triazin-hexahydrats.

Während TMT-H₃ praktisch nicht (unter 0,5 Gew.-%) und TMT-Na · 3 H₂O wenig (etwa 3 Gew.-%, berechnet als TMT-Na) in Wasser löslich ist, zeigt TMT-Na₃ · 9 H₂O eine erwartungsgemäß gute Löslichkeit (bei 0 °C etwa 16 Gew.-%, berechnet als TMT-Na₃). Daß die Löslichkeit von TMT-Na₂ · 6 H₂O, wie nun gefunden wurde, wesentlich über derjenigen des an sich gut löslichen TMT-Na₃ · 9 H₂O liegt, konnte nicht erwartet werden. Während eine an TMT-Na₃ · 9 H₂O gesättigte wäßrige Lösung bei 0 °C 0,78 Mol TMT-Na₃ pro l Wasser enthält, liegt der Gehalt einer an TMT-Na₂ · 6 H₂O gesättigten wäßrigen Lösung bei 0 °C bei etwa 2 Mol TMT-Na₂ pro l Wasser.

TMT-Na₂ · 6 H₂O weist mit einem Gehalt von 67 Gew.-% TMT-Na₂ nicht nur einen höheren Wirkstoffgehalt auf als TMT-Na₃ · 9 H₂O, sondern das erfindungsgemäße Salz gestattet zudem die Herstellung konzentrierterer wäßriger Lösungen, welche bei niedrigen Temperaturen lagerstabil sind, also nicht auskristallisieren, als dies nach dem Stand der Technik möglich war. Der eigentliche Wirkstoff der Natriumsalze von TMT-H₃ ist das Trimercapto-s-triazin, so daß ein höherer Gehalt im Feststoff (Mol TMT-Salz / kg) als auch in den daraus zugänglichen Lösungen zu erheblichen Kosteneinsparungen beim Transport und der Lagerung führt. Ein weiterer Vorteil von TMT-Na₂ · 6 H₂O besteht in seiner geringeren Alkalität.

TMT-Na₂ · 6 H₂O kann als Fällungsmittel zur Abtrennung von Schwermetallen aus wäßriger Phase, insbesondere aus Abwässern oder Prozeßwässern, verwendet werden. Die Fällung der schwerlöslichen Schwermetallsalze des TMT-H₃ erfolgt in an sich bekannter Weise - in Analogie zur Verwendung der vorbekannten TMT-Na-Salze bzw. deren wäßrigen Lösungen. Meist erfolgt die Behandlung der wäßrigen Phase im pH-Bereich 8-10. TMT-Na₂ führt, sofern nach der Schwermetallabtrennung rückneutralisiert werden muß, zu einem geringeren Neutralsalzanfall als das bisher fast ausschließlich in der Praxis verwendete TMT-Na₃.

Gegenstand der Erfindung ist auch ein Verfahren zur Herstellung von TMT-Na₂ · 6 H₂O, das dadurch gekennzeichnet ist, daß man das Trinatriumsalz des Trimercapto-s-triazin-nonahydrats (TMT-Na₃ · 9 H₂O) mit Trimercapto-s-triazin (TMT-H₃) im Molverhältnis 2 - 2,1 : 1 bei 30-60 °C in wäßrigem Medium miteinander umsetzt, indem man unter intensivem Mischen TMT-Na₃ · 9 H₂O und TMT-H₃ in einer solchen Menge in Wasser einträgt, daß eine an gebildetem Dinatriumsalz des Trimercapto-s-triazins etwa gesättigte Lösung entsteht, anschließend auf 0-10 °C abkühlt, das auskristallisierte TMT-Na₂ · 6 H₂O von der Mutterlauge abtrennt und unter schonenden Bedingungen trocknet.

Vorzugsweise setzt man TMT-Na₃ · 9 H₂O gegenüber dem stöchiometrischen Molverhältnis von 2:1 in geringem Überschuß ein. Sofern die Ausgangsstoffe selbst aus Cyanurchlorid und NaSH oder Na₂S oder NaSH/Na₂S in wäßrigem Medium hergestellt wurden, ist es wirtschaftlicher, anstelle getrockneter TMT-Ausgangsprodukte diese in filterfeuchter Form einzusetzen.

Filterfeuchtes TMT-Na₂ · 6 H₂O verliert bei der Lagerung an der Luft solange Restfeuchtigkeit durch Verdampfung, bis der TMT-Na₃-Gehalt ca. 67 Gew.-% und der Kristallwassergehalt ca. 33 Gew.-% beträgt. Die Trocknung kann bei 20-30 °C auch unter vermindertem Druck und/oder über Trocknungsmitteln erfolgen.

Die Struktur von TMT-Na₂ · 6 H₂O wurde mittels folgender Methoden abgesichert: 1. Identifikation durch Ansäuern und Vergleich mit Muster TMT-H₃; 2. Gehaltsbestimmung durch Säure-Base-Titration; 3. Reinheitsbestimmung durch HPLC; 4. Wasserbestimmung. Beim Erwärmen des TMT-Na₂ · 6 H₂O wird ab 35 °C Kristallwasser abgespalten. Die Abspaltung erfolgt in mehreren Stufen und ist bei 149 °C beendet (Thermogravimetrie (TG) und Dynamische Differential Kalorimetrie (OSC)).

Zwar ist es möglich, kristallines TMT-Na₂ · 6 H₂O in vergleichbarer Weise direkt aus Cyanurchlorid, NaSH und NaOH herzustellen, wie dies für TMT-Na₃ · 9 H₂O in der DE-OS 37 29 029 beschrieben wird, dies ist aber weniger vorteilhaft. Anders als TMT-Na₃ · 9 H₂O ist TMT-Na₂ · 6 H₂O aus dem kochsalzhaltigen Reaktionsgemisch schlechter filtrierbar, weshalb hohe Restfeuchten, welche zusätzlich aus der Herstellung stammendes Kochsalz enthalten, am Produkt verbleiben. Ferner enthält die Mutterlauge nach der Kristallisation von TMT-Na₂ · 6 H₂O wegen dessen hoher Wasserlöslichkeit noch erhebliche Mengen dieses Salzes, das durch Ausfällung als TMT-H₃ gewonnen werden muß.

Durch die nachstehend aufgezeigten Veränderungen des in der genannten DE-OS 37 29 029 beschriebenen Herstellverfahrens für TMT-Na₃ · 9 H₂O aus Cyanurchlorid konnten sowohl erfindungsgemäßes kristallines TMT-Na₂ · 6 H₂O als auch konzentrierte Lösungen dieses Salzes in einfacher Weise und praktisch kochsalzfrei zugänglich gemacht werden.

Diese Veränderungen bestehen darin, daß nach beendeter Umsetzung des Cyanurchlorids mit NaSH oder NaSH/Na₂S und NaOH die Kristallisationsbedingungen für TMT-Na₃ · 9 H₂O durch Erhöhung der Temperatur und/oder Zugabe von Wasser und/oder Einstellung eines niedrigeren pH-Wertes so eingestellt werden, daß nur ein Teil, vorzugsweise etwa zwei Drittel, der gebildeten TMT-Na-Salze als TMT-Na₃ · 9 H₂O auskristallisieren. Nach Abtrennen des Salzes und ggf. Nachwaschen mit Wasser, wird aus den vereinigten Filtraten -Mutterlauge und Waschwasser - durch Zugabe einer Mineralsäure, vorzugsweise Salzsäure, TMT-H₃ ausgefällt, dieses abgetrennt und ggf. gewaschen. Der feuchte Filterkuchen TMT-Na₃ · 9 H₂O und der feuchte Filterkuchen TMT-H₃ werden im Molverhältnis von etwa 2 - 2,1 : 1 bei 40-50 °C in wenig Wasser eingetragen und miteinander umgesetzt. Vorzugsweise trägt man eine solche Menge TMT-Na₃ · 9 H₂O und TMT-H₃ in Wasser ein, bis die Sättigungsgrenze an TMT-Na₂ etwa erreicht ist und kühlt dann zur Kristallisation des TMT-Na₂ · 6 H₂O ab. Sofern zunächst nur eine an TMT-Na₂ noch nicht gesättigte Lösung vorliegt, kann diese durch Abdestillieren von Wasser unter vermindertem Druck bei mäßiger Temperatur vor dem Kristallisieren des TMT-Na₂ · 6 H₂O konzentriert werden.

Der besondere Vorteil dieser erfindungsgemäßen Ausführungsform der Herstellung des erfindungsgemäßen Produktes besteht darin, daß nur maximal 33 % des aus Cyanurchlorid gebildeten Trimercapto-s-triazins als TMT-H₃ zwischenisoliert werden müssen, das Verfahren einfacher durchführbar ist und praktisch kochsalzfreie Produkte erhalten werden.

### Beispiel 1

172 g 40 gew.-%ige wäßrige NaSH (1,23 mol) werden mit 112 ml Wasser verdünnt. Dazu werden bei 50 °C 75 g Cyanurchlorid (0,41 mol) und 60 g 50 gew.-%ige wäßrige NaOH (0,75 mol) so dosiert, daß der pH-Wert zwischen 9,5 und 10,5 und die Temperatur bei 50 °C gehalten wird. Nach beendeter Zugabe wird noch 1 h bei 50 °C gerührt und anschließend durch Zufügen von 39 g 50 gew.-%iger wäßriger NaOH (0,48 mol) der pH-Wert auf 12,5 gestellt. Nach dem Kühlen auf 10 °C wird zentrifugiert und der Filterkuchen mit 50 ml 20 °C warmen Wasser auf der Zentrifuge gewaschen.

Der Feststoff wiegt 119,2 g und enthält neben Wasser und Spuren Kochsalz (weniger als 0,3 Gew.-%) 54,1 Gew.-% TMT-Na₃. Filtrat und Waschwasser werden vereinigt und mit 50 g konzentrierter Salzsäure angesäuert, und das dabei quantitativ ausfallende TMT-H₃ filtriert und mit Wasser kochsalzfrei gewaschen. Der gewaschene TMT-H₃-Filterkuchen wiegt 59,0 g und enthält 34,3 Gew.-% TMT-H₃.

Die Filterkuchen werden in 120 ml Wasser eingerührt. Es entstehen 298 g einer Lösung, in der 0,38 mol TMT-Na-Salze gelöst sind. Dies entspricht einer Ausbeute von 93,3 %, bezogen auf eingesetztes Cyanurchlorid. Am TMT sind 2,09 Wasserstoff gegen Natrium ersetzt. Es handelt sich um eine ca. 28 gew.-%ige TMT-Na₂-Lösung.

### Beispiel 2

296 g der gemäß Beispiel 1 erhaltenen Lösung werden im Vakuum bis 50 °C eingeengt, bis 115 ml Wasser abdestilliert sind. Beim Abkühlen auf 10 °C kristallisiert Feststoff aus, der durch Filtration abgetrennt wird. Isoliert werden 129 g Feststoff, der 51,5 Gew.-% TMT-Na₂ und 48,5 Gew.-% H₂O (freies und als Kristallwasser gebundenes H₂O) enthält, sowie 51 g Filtrat, das 34 Gew.-% TMT-Na₂ enthält. Durch vorsichtiges Trocknen des Feststoffs an der Luft erhält man TMT-Na₂ · 6 H₂O.

### Beispiel 3

90 g filterfeuchtes TMT-Na₃ · 9 H₂O (54 Gew.-% TMT-Na₃) (=0,2 Mol) und 50 g filterfeuchtes TMT-H₃ (34 Gew.-% TMT-H₃) (=0,096 Mol) werden unter Rühren und Aufrechterhaltung einer Temperatur von 50 °C und des Molverhältnisses 2:1 portionsweise in 20 ml Wasser eingetragen. Man erhält eine hochkonzentrierte, praktisch klare TMT-Na₂-Lösung, welche man anschließend auf etwa 5 °C abkühlt, wobei TMT-Na₃ · 6 H₂O auskristallisiert. Nach dem Abtrennen des auskristallisierten Produktes durch Filtrieren oder Zentrifugieren kann die Mutterlauge erneut für die Umsetzung verwendet werden.

### Beispiel 4

15 g Na₂S (0,19 mol) und 48 g NaHS (0,8 mol) werden in 133 ml Wasser vorgelegt und bei 40 °C 65 g Cyanurchlorid (0,35 mol) so zugefügt, daß die Temperatur gehalten wird. Der pH-Wert sinkt zunächst auf 9 und wird dort durch gleichzeitige Zugabe einer 30 gew.-%igen wäßrigen NaOH gehalten, bis sämtliches Cyanurchlorid zugefügt ist. Nach einer halben Stunde Nachreaktionszeit wird der pH-Wert durch Zufügen des Restes der insgesamt 115 g Natronlauge (0,86 mol) auf über 12,5 erhöht. Die Aufarbeitung erfolgt wie gewohnt, jedoch wird bei 30 °C kristallisiert und der TMT-Na₃-Filterkuchen nicht gewaschen.

Man erhält 101 g Filterkuchen, der 52,3 Gew.-% TMT-Na₃, 1,5 Gew.-% Kochsalz und 46,2 Gew.-% Wasser enthält, sowie 45 g Filterkuchen, der 35,2 Gew.-% TMT-H₃ und 64,8 Gew.-% Wasser enthält. Durch Umsetzung der Filterkuchen in das Molverhältnis 2:1 gemäß Beispiel 3 erhält man TMT-Na₂ · 6 H₂O.

## Patentansprüche

1. Dinatriumsalz des Trimercapto-s-triazin-hexahydrats (TMT-Na₂ · 6 H₂O).

2. Verfahren zur Herstellung der Verbindung gemäß Anspruch 1,
dadurch gekennzeichnet,
daß man das Trinatriumsalz des Trimercapto-s-triazin-nonahydrats (TMT-Na₃ · 9 H₂O) mit Trimercapto-s-triazin (TMT-H₃) im Molverhältnis 2 - 2,1 : 1 bei 30-60 °C in wäßrigem Medium miteinander umsetzt, indem man unter intensiven Mischen TMT-Na₃ · 9 H₂O und TMT-H₃ in einer solchen Menge in Wasser einträgt, daß eine an gebildetem Dinatriumsalz des Trimercapto-s-triazins etwa gesättigte Lösung entsteht, anschließend auf 0-10 °C abkühlt, das auskristallisierte TMT-Na₂ · 6 H₂O von der Mutterlauge abtrennt und unter schonenden Bedingungen trocknet.

3. Verwendung der Verbindung gemäß Anspruch 1 als Fällungsmittel zur Abtrennung von Schwermetallen aus wäßriger Phase.

## Claims

1. The disodium salt of trimercapto-s-triazine hexahydrate (TMT-Na₂.6H₂O).

2. A process for the preparation of the compound according to Claim 1,
characterised in that
the trisodium salt of trimercapto-s-triazine nonahydrate (TMT-Na₃.9H₂O) and trimercapto-s-triazine (TMT-H₃) are made to react with each other in the molar ratio of 2 - 2.1 : 1 at 30 - 60°C in aqueous medium, by introducing TMT-Na₃.9H₂O and TMT-H₃, with intensive mixing, into an amount of water such that an approximately saturated solution of the disodium salt of trimercapto-s-triazine is produced, then cooling to 0 - 10°C, separating the crystallised TMT-Na₂.6H₂O from the mother liquor and drying under mild conditions.

3. Use of the compound according to Claim 1 as a precipitating agent to separate heavy metals from an aqueous phase.

## Revendications

1. Sel disodique de trimercapto-s-triazine hexahydrate.

2. Procédé de préparation du composé selon la revendication 1, caractérisé en ce qu'on fait réagir le sel trisodique de la trimercapto-s-triazine nonahydrate (TMT-Na₃ · 9H₂O) avec de la trimercapto-s-triazine (TMT-H₃) en proportion molaire 2-2,1 : 1 entre 30 et 60°C dans un milieu aqueux, en incorporant à l'eau sous agitation énergique TMT-Na₃ · 9H₂O et TMT-H₃ en quantité telle qu'il se forme une solution approximativement saturée en sel disodique formé de la trimercapto-s-triazine, ensuite on refroidit entre 0 et 10°C, on sépare le TMT-Na₂ · 6H₂O qui a recristallisé de la solution mère et on sèche sous des conditions modérées.

3. Utilisation du composé selon la revendication 1 comme agent de précipitation pour séparer des métaux lourds de la phase aqueuse.
